# EUROPEAN PATENT APPLICATION

(11) **EP 4 768 066 A1**
(43) Date of publication of application: **01.07.2026**
(21) Application number: 24954599.7
(22) Date of filing: 30.09.2024
(51) Int. Cl.: A61M 25/10, A61B 17/22

(54) **SHOCKWAVE BALLOON CATHETER WITH EXTERNAL GUIDE WIRE**

(30) Priority: 29.09.2024 CN 202411367631
(71) Applicant: Vascupatent Medical (Shenzhen) Co., Ltd., Shenzhen, Guangdong 518122 (CN)
(72) Inventor: OUYANG, Junxiong, Shenzhen, Guangdong 518122 (CN); LU, Lizhong, Shenzhen, Guangdong 518122 (CN)
(74) Representative: Tahtadjiev, Konstantin
(86) International application number: PCT/CN2024/122604
(87) International publication number: WO 2026/065348

(57) **Abstract**

This invention discloses an external guidewire shockwave balloon catheter including a balloon, a catheter, and a guidewire. The catheter includes an inner support rod and an outer tube that are deformable for supporting the balloon. The inner support rod is located inside the outer tube and is coaxial, with a gap between the outer tube and the inner support rod. The inner cavity of the balloon is communicated with the gap to allow the introduction of electrolyte fluid. The distal end of the inner support rod extends out from the distal end of the outer tube, and the proximal end of the balloon is sealed to the distal end of the outer tube. The distal end of the balloon has a balloon tip, which is sealed to the distal end of the inner support rod. A corona generator is located at the position of the inner support rod where it is positioned within the balloon, and an inlet communicating with the guidewire hole is located at the balloon tip. Compared with conventional technologies, by placing the external guidewire at the balloon tip, this invention achieves greater stress concentration on the vascular wall while guiding the balloon into the blood vessel and reaching the lesion site, and also enables the guidewire to function as a cutting guidewire.

## Description

### BACKGROUND OF THE PRESENT INVENTION

### FIELD OF INVENTION

The present invention relates to a medical device, and more particularly to an external guidewire shockwave balloon catheter based on liquid corona discharge.

### DESCRIPTION OF RELATED ARTS

In the field of medical devices, balloon catheters are mainly used to dilate stenosed blood vessels, and this treatment method has been proven to be sufficiently safe and effective. At present, balloon catheters mainly expand the balloon based on the principle of arc discharge. Arc discharge is a discharge phenomenon in which a high-density plasma channel is formed through a medium under high voltage. Under high-voltage conditions, the medium between electrodes is completely broken down, forming a continuous high-temperature and high-density plasma channel. Arc discharge is a strong discharge phenomenon characterized by high temperature and high current density. It is an overall effect in which the arc penetrates the medium between electrodes to form a continuous high-temperature and high-current channel. Under a sufficiently high electric field, a liquid medium is completely broken down to form a high-density plasma channel, and this process is accompanied by intense energy release and high temperature generation. The shock wave energy generated by arc discharge is enormous and difficult to control precisely, which may cause unpredictable damage to surrounding tissues. Although the strong shock wave effect of arc discharge can rapidly fracture calcified plaques, it also exerts a significant impact on surrounding vascular tissues, potentially leading to vascular injury or other complications. The high-energy shock waves generated by arc discharge may result in high-risk complications such as vascular rupture and thrombosis. During the arc discharge process, the high temperature in the arc region may spread to surrounding tissues, causing unnecessary thermal damage. Moreover, the shock wave effect of arc discharge is typically strong in a single instance, making it difficult to achieve multiple precise controls and cumulative therapeutic effects. In the prior art, the fragmentation of calcification mainly relies on the water hammer effect generated at the moment when the balloon expands outward. Since the balloon is generally made of a polymer film material with relatively low hardness, and because a guide wire is built into the inner tube of the balloon, the outer diameter of the balloon tends to be relatively large. When entering a calcified vascular site, the current generated after pulse excitation is relatively small, and the outward expansion force may be insufficient. Therefore, during clinical treatment, the catheter may have insufficient capability to fracture calcified lesions.

### SUMMARY OF THE PRESENT INVENTION

The present invention is to provide an externally mounted shockwave balloon catheter. The technical problem to be solved is to reduce the overall outer diameter of the balloon catheter and to create stress concentration on the externally mounted guidewire, thereby increasing the pulse energy and enabling the guidewire to perform a cutting action.

To solve the above problems, the present invention adopts the following technical solution: An externally guidewire shockwave balloon catheter, comprising a balloon, a catheter, and a guidewire, wherein the catheter comprises an inner support rod and an outer tube that are deformable and arranged to support the balloon, wherein the inner support rod is located inside the outer tube and is coaxial with the outer tube, a gap is provided between the outer tube and the inner support rod, an inner cavity of the balloon is communicated with the gap to allow the introduction of electrolyte liquid, wherein a distal end of the inner support rod is extended out from a distal end of the outer tube, a proximal end of the balloon is sealed to the distal end of the outer tube, a distal end of the balloon is provided with a balloon tip, the balloon is sealed to the distal end of the inner support rod through the balloon tip, a corona generator is provided at a position of the inner support rod located at the balloon to achieve corona discharge to cause the electrolyte liquid in the balloon to have a corona reaction, so as to generate steam bubbles to expand the balloon, wherein a guide wire hole is provided at a distal end of the balloon tip to penetrate the distal end of the balloon tip, the guide wire hole is not communicated to the inner cavity of the balloon, wherein an inlet is provided on the balloon tip to communicate with the guide wire hole so as to allow the guide wire to be inserted into the guide wire hole through the inlet and then extended out from the distal end of the balloon tip, so that a part of the guide wire is placed outside the balloon and the catheter, in addition to guiding the balloon, the guide wire also serves as a cutting guide wire to assist in cutting the lesion site after the electrolyte liquid has a corona reaction.

Furthermore, a diameter of the inner support rod is less than or equal to a diameter of the inlet, and the diameter of the inner support rod is less than or equal to a diameter of the guidewire hole.

Furthermore, the corona generator comprises at least one set of electrode group which is provided with discharge regions, where each set of electrodes comprises a first electrode connected to a positive lead and a second electrode connected to a negative lead, wherein the first electrode and the second electrode in the electrode group are spaced apart, the first electrode and the second electrode are provided with discharge regions that are in contact with the electrolyte liquid filled in the inner cavity of the balloon, so as to allow an electric field to be formed between the first electrode and the second electrode, wherein the first electrode and the second electrode are fixed on the inner support rod.

Furthermore, the positive and negative leads are respectively provided with exposed areas connected to the first electrode and the second electrode, and the exposed areas are covered with metal electrode sleeves which are respectively connected and fixed to the first electrode and the second electrode.

Furthermore, each of the first electrode and the second electrode has a ring shape, and a groove is respectively formed on an edge of an inner wall of each of the first electrode and the second electrode, and a metal electrode sleeve is fixed on the groove.

Furthermore, outer walls of the first electrode and the second electrode are respectively provided with a first insulating layer and a second insulating layer at other positions except for the discharge regions, so as to expose the discharge region.

Furthermore, the first electrode is provided with at least one first through hole, the second electrode is provided with at least one second through hole, the at least one first through hole and the at least one second through hole respectively constitute the discharge regions, the first insulating layer is completely covered on the rest position of the first electrode except for the at least one first through hole, and the second insulating layer is completely covered the rest position of the second electrode except for the at least one second through hole, so as to form a directional electric field between the first electrode and the second electrode.

Furthermore, the at least one set of electrode group comprise a single electrode group which comprises one first electrode and one second electrode.

Furthermore, the at least one set of electrode group comprise a single electrode group which comprises two first electrodes and one second electrode, wherein the second electrode is disposed between the two first electrodes.

Furthermore, the at least one set of electrodes comprise two sets of electrodes, wherein one electrode set comprises two first electrodes and one second electrode, and the other electrode set comprises one first electrode and one second electrode.

Compared with the prior art, the present invention has the following advantages:

1. By setting an external guidewire at the tip of the balloon, the balloon is guided into the blood vessel and reaches the lesion site. At the same time, the corona generator receives the pulse signal sent by the power pulse generator. Under the action of the pulse signal, the electrolyte liquid undergoes a corona reaction, and the electrolyte liquid produces molecular ionization. The vapor bubbles generated by molecular ionization compress the electrolyte liquid, increasing the pressure inside the balloon. This causes the balloon to expand radially along the inner tube, allowing the guidewire to adhere tightly to the blood vessel wall. This makes the stress of the guidewire on the inner wall of the blood vessel more concentrated and enables the guidewire to function as a cutting guidewire.

2. At the same time, external guidewire placement can increase the electrolyte capacity inside the balloon, thereby reducing the resistance inside the balloon, increasing the pulse current, increasing the size of the vapor bubble, and enhancing the outward expansion force.

3. The present invention also reduces the overall outer diameter of the balloon. The outer diameter of the balloon in the folded state is mainly determined by the outer diameter of the inner support tube. However, since the inner support rod of the present invention does not have a guide wire through hole, the outer diameter of the inner support tube can be reduced to more than 1/2 of that of the inner tube of a typical balloon in the prior art. Therefore, the balloon can reach a more distal end of the vascular lesion.

### BRIEF DESCRIPTION OF THE DRAWINGS

FIG. 1 is a schematic view of the structure of the present invention.
FIG. 2 is a cross-sectional view along the A-A direction in FIG. 1.
FIG. 3 is a cross-sectional view along the B-B direction in FIG. 1.
FIG. 4 is a partial enlarged view of the part marked C in FIG. 1.
FIG. 5 is a schematic view of the electrode assembly of the present invention.
FIG. 6 is another schematic view of the electrode assembly of the present invention.
FIG. 7 is a schematic view of the electric field of the electrode assembly of the present invention.
FIG. 8 is a schematic view of the structure of the present invention with two electrodes.
FIG. 9 is a circuit diagram of the present invention with two electrodes.
FIG. 10 is a schematic view of the structure of the present invention with three electrodes.
FIG. 11 is a circuit diagram of the present invention with three electrodes.
FIG. 12 is a schematic view of the structure of the two sets of electrodes of the present invention.
FIG. 13 is a circuit diagram of the two sets of electrodes of the present invention.
FIG. 14 is a schematic view of the present invention entering a blood vessel.
FIG. 15 is a schematic view of the corona discharge of the present invention.
FIG. 16 is a schematic view illustrating the guidewire of the present invention breaking up the lesion.

### DETAILED DESCRIPTION OF THE PREFERRED EMBODIMENT

The present invention will now be described in further detail with reference to the accompanying drawings and embodiments.

In the present invention, the distal end refers to the end furthest from the surgeon; the proximal end refers to the end closest to the surgeon.

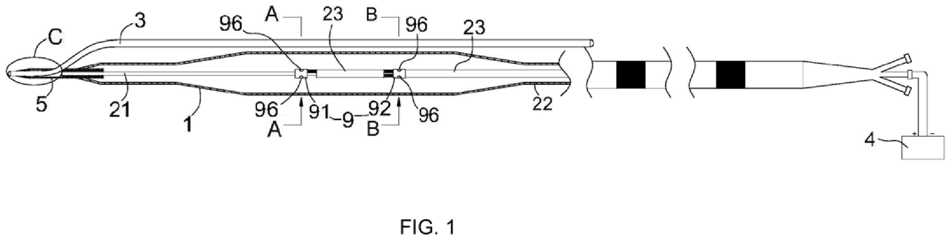

As shown in FIGS. 1 to 4, the present invention discloses an externally mounted shockwave balloon catheter comprising an expandable balloon 1, a catheter 2, and a guidewire 3.

The catheter 2 comprises an inner support rod 21 and an outer tube 22 that are elastically deformable and used to support the balloon 1. The inner support rod 21 is located inside the outer tube 22 and is coaxial. There is a gap between the outer tube 22 and the inner support rod 21. The inner cavity of the balloon 1 is communicated with the gap to allow electrolyte liquid to be introduced. The distal end of the inner support rod 21 is extended out from the distal end of the outer tube 22.

The proximal end of the balloon 1 is sealed to the distal end of the outer tube 22. The distal end of the balloon 1 is provided with a balloon tip 5. The balloon 1 is sealed to the distal end of the inner support rod 21 through the balloon tip 5.

A corona generator is provided at the position of the inner support rod 21 in the balloon 1 to realize the corona reaction of the electrolyte liquid in the balloon 1 through corona discharge, generate steam bubbles to expand the balloon 1. A guide wire hole 7 is provided at the distal end of the balloon tip 5, which penetrates the distal end of the balloon tip 5. The guide wire hole 7 is not communicated to the inner cavity of the balloon 1. An inlet 6 communicated to the guide wire hole 7 is provided on the balloon tip 5.

After the guidewire 3 is inserted into the guidewire hole 7 through the inlet 6, it extends from the distal end of the balloon tip 5, so that a part of the guidewire 3 is external to the balloon 1 and the catheter 2. In addition to guiding the balloon 1, the guidewire 3 also acts as a cutting guidewire to assist in cutting the lesion after the corona reaction of the electrolyte liquid occurs. Because the external guidewire has a certain degree of hardness and toughness, its hardness can cut calcified tissue to a certain extent, while its toughness ensures that it will not easily break when the cutting force is applied. After the present invention is delivered to the lesion site through the external guidewire, the guidewire can accurately transmit the mechanical force generated after the pulse to the cut tissue. The operator controls the pushing and rotating movements of the guidewire to make relative movement between the guidewire and the calcified tissue, thereby achieving the cutting effect.

In the present invention, the inner support rod 21 can be a solid round rod or a round rod with a central hole. The inner support rod 21 is made of polymer materials, such as Nylon, PI ( polyimide ), or Pebax (polyether block polyamide).

The diameter of the inner support rod 21 is less than or equal to the diameter of the inlet 6 and the guide wire hole 7.

In the present invention, the corona generator can be implemented using the following structure, as shown in FIGS. 1 to 3, the corona generator comprises at least one set of electrode groups 9. Each set of electrode groups 9 comprises at least one first electrode 91 connected to a positive lead and at least one second electrode 92 connected to a negative lead. The positive and negative leads are electrically connected to a conventional power pulse generator 4 via electrical connectors. The first electrode 91 and the second electrode 92 in the electrode group 9 are spaced apart. Each of the first electrode 91 and the second electrode 92 is provided with a discharge region 96 which is a contact point that contacts the electrolyte liquid filled in the cavity of the balloon 1. The discharge region 96 is used to form an electric field between the first electrode 91 and the second electrode 92. The corona generator receives the pulse electrical signal sent by the power pulse generator 4. Under the action of the pulse electrical signal, the electrolyte liquid undergoes a corona discharge through the discharge regions 96, and the electrolyte liquid produces molecular ionization. The vapor bubbles generated by the molecular ionization form at the position of the discharge regions 96 and squeeze the electrolyte liquid, increasing the pressure inside the balloon 1. This causes the balloon 1 to expand radially along the inner support rod 21. The first electrode 91 and the second electrode 92 are fixed to the inner support rod 21 by pasting or welding.

As shown in FIG. 1, a PET heat shrink tubing 23 can be fitted between the inner support rod 21 and the lead wires to fix the lead wires to the inner support rod 21. Alternatively, the inner support rod 21 and the wire can be integrally formed by coextrusion.

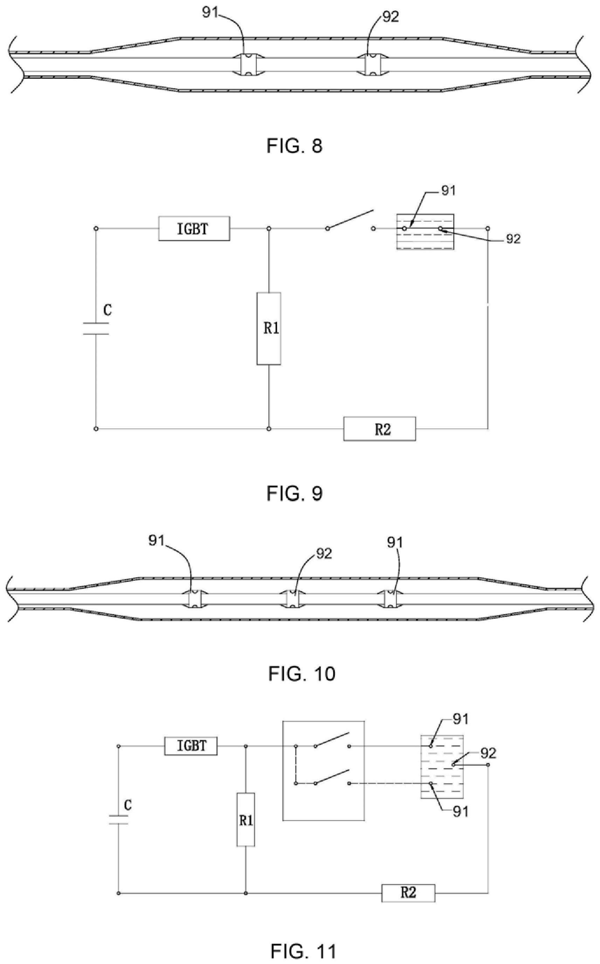

When in use, the corona generator is placed in an electrolyte liquid. The corona generator receives a pulse signal from the power pulse generator 4, causing a corona reaction in the electrolyte liquid, which ionizes the liquid molecules. The plasma generated by the molecular ionization vaporizes the surrounding liquid at high temperature, producing vapor bubbles. As the ionization reaction continues, the vapor bubbles expand and compress the surrounding liquid environment. Due to the incompressible nature of the liquid, the internal pressure of the balloon 1 increases instantaneously, causing the balloon 1 to expand radially (as shown in FIG. 11) until the ionization reaction ends and the balloon recovers. The duration of the ionization reaction is determined by the pulse signal duration and pulse width parameters.

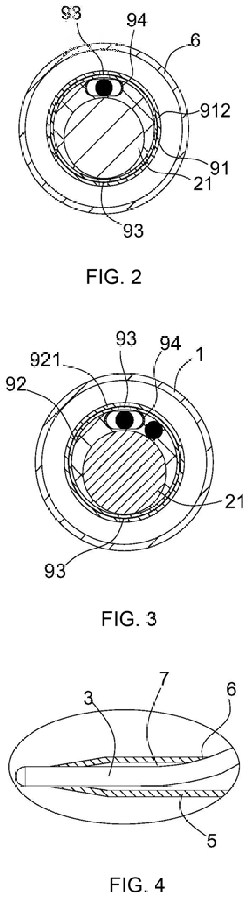

As shown in FIGS. 2, 3, 6, and 7, exposed areas are provided on the positive and negative leads, which are connected to the first electrode 91 and the second electrode 92. The remaining areas of the positive and negative leads are covered with insulating layers. A metal electrode sleeve 94 can be fitted over the exposed area of each of the positive and negative conductors along the inner tube. The metal electrode sleeve 94 is flat and is connected and fixed to the exposed area by pasting or pressing, so that the metal electrode sleeve 94 is tightly connected to the exposed area. The metal electrode sleeve 94 is connected and fixed to each of the first electrode 91 and the second electrode 92 by welding.

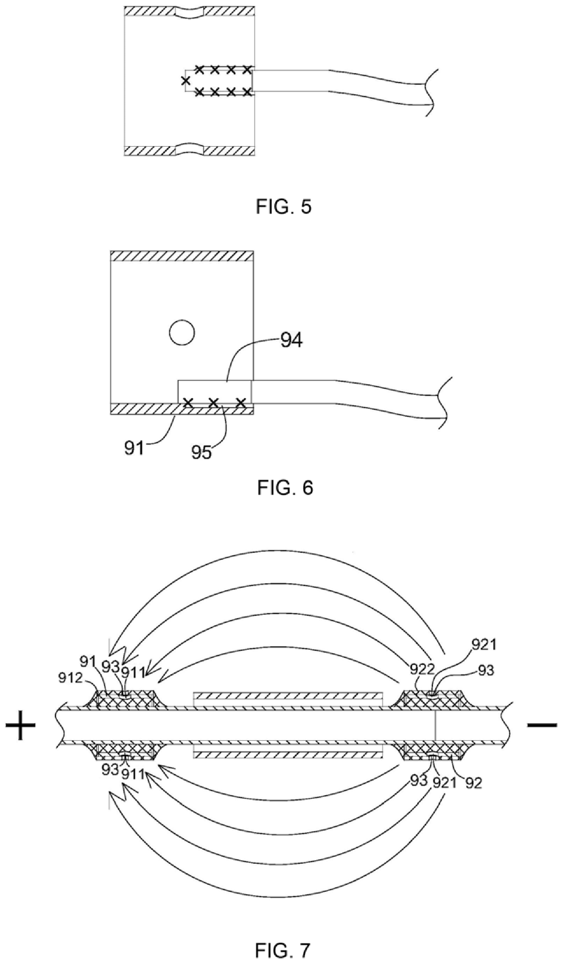

As shown in FIGS. 5 and 6, each of the first electrode 91 and the second electrode 92 in the present invention is ring-shaped, specifically circular. FIGS. 5 and 6 illustrate the first electrode 91 as an example. Grooves 95 are respectively formed on the edges of the inner wall of the first electrode 91 and the second electrode 92. The metal electrode sleeve 94 is welded to the corresponding groove 95. Specifically, the overall size of the groove 95 can be smaller than the overall size of the metal electrode sleeve 94, so as to form a contact position on each of the first electrode 91 and the second electrode 92 that can be welded to the metal electrode sleeve 94, as shown in the " X " positions in FIGS. 5 and 6 in which the metal electrode sleeve 94 is shown to be connected to each of the first electrode 91 and the second electrode 92.

In the present invention, as shown in FIGS. 2 and 3, the outer walls of the first electrode 91 and the second electrode 92 are respectively provided with a first insulating layer 912 and a second insulating layer 922 at positions other than the discharge region 96, so as to expose the discharge region 96 and insulate the other positions, thereby receiving the pulse electrical signal sent by the power pulse generator 4 at the electrodes and forming a directional electric field between the electrodes.

As shown in FIG. 7, the electrode assembly structure of the present invention has at least one first through hole 911 on the first electrode 91 and at least one second through hole 921 on the second electrode 92. The first through hole 911 and the second through hole 921 respectively constitute discharge regions 96. The first insulating layer 912 completely covers the first electrode 91 except for the first through hole 911, and the second insulating layer 922 completely covers the second electrode 92 except for the second through hole 921. This allows the first electrode 91 and the second electrode 92 to receive pulsed electrical signals sent by the power pulse generator 4 and form a directional electric field between the electrodes. Under the action of the pulsed electrical signals, the electrolyte liquid undergoes a corona reaction. The potential distribution direction of this electrode assembly structure is relatively concentrated, which helps to reduce heat generation. The electron passage path is more concentrated, the resistance is higher, the current is reduced, and the safety is increased. It also facilitates steady-state symmetrical pressurization inside the balloon, allowing the balloon to expand uniformly outward. The first insulating layer 912 and the second insulating layer 922 can reduce the contact area between the first electrode 91, the second electrode 92, and the electrolyte liquid, allowing charge release in the controlled discharge regions 96.

The first through holes 911 are symmetrically arranged on the first electrode 91, and the second through holes 921 are symmetrically arranged on the second electrode 92. The two first through holes 911 and the two second through holes 921 are respectively arranged in a one-to-one correspondence. The metal electrode sleeve can be arranged at any position on the inner ring wall of each of the first electrode 91 and the second electrode 92.

The distance between the first through hole 911 on the first electrode 91 and the second through hole 921 on the second electrode 92 is 3-8 mm.

As can be seen from FIG. 7, the electric field formed in this embodiment is formed between the two through holes of the two electrodes, and the electric field is formed in two regions from the second through hole 921 of the second electrode 92 toward the first through hole 911 of the first electrode 91. Under the action of the pulsed electric signal, the electrolyte liquid undergoes a corona reaction.

When the first electrode group forms an electric field, the generated vapor bubbles are distributed at the positions of the first through hole 911 and the second through hole 921.

In the present invention, the electrode assembly is made of high-temperature resistant materials such as 304 stainless steel, 316 stainless steel, or tungsten-containing alloys. While ensuring excellent conductivity, the material can withstand the high-temperature plasma thermal erosion generated during corona discharge. The electrode assembly is spaced to prevent arcing between electrodes, which could lead to breakdown discharge. This space needs to be filled with electrolyte liquid to form a conductive path. Under certain parameters, a longer spacing results in a higher resistance in the conductive path and a smaller current.

As shown in FIGS. 8 and 9, when the corona generator comprises one set of electrodes, the electrode set comprises one first electrode 91 and one second electrode 92. The first electrode 91 is connected to the positive terminal of the power pulse generator 4, and the second electrode 92 is connected to the negative terminal of the power pulse generator 4. The two electrodes receive the pulse electrical signal from the power pulse generator 4 and form an electric field between the discharge regions of the electrodes. Under the action of the pulse electrical signal, the electrolyte liquid undergoes a corona reaction.

As shown in FIGS. 10 and 11, when the corona generator comprises one set of electrodes which comprises two first electrodes 91 and one second electrode 92. The second electrode 92 is disposed between the two first electrodes 91 with equal spacing. The two first electrodes 91 are connected to the positive terminal of the power pulse generator 4, and the second electrode 91 is connected to the negative terminal of the power pulse generator 4. The two first electrodes 91 and the second electrode 92 receive the pulse electrical signal sent by the power pulse generator 4 and form an electric field between the discharge regions of each group of two adjacent electrodes. Under the action of the pulse electrical signal, the electrolyte liquid undergoes a corona reaction.

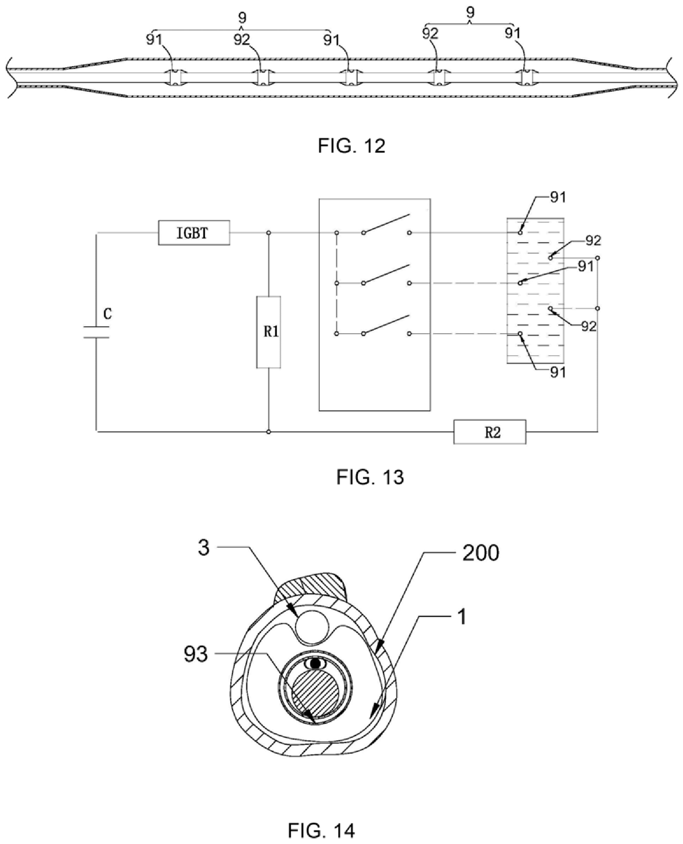

As shown in FIGS. 12 and 13, when the corona generator comprises two sets of electrodes, one set of electrodes comprises two first electrodes 91 and one second electrode 92, and the other set of electrodes comprises one first electrode 91 and one second electrode 92. In the set of electrodes 9 with three electrodes, the second electrode 92 is located between the two first electrodes 91, and the electrodes of the two sets of electrodes are arranged at equal distances. The three first electrodes 91 are connected to the positive terminal of the power pulse generator 4, and the two second electrodes 92 are connected to the negative terminal of the power pulse generator 4. The two sets of electrodes 9 receive the pulse electrical signals sent by the power pulse generator 4 and form an electric field between their respective electrodes, causing the electrolyte liquid to undergo a corona reaction under the action of the pulse electrical signals. Preferably, the first electrodes 91 and the second electrodes 92 in the two sets of electrodes are arranged alternately.

As shown in FIGS. 9, 11, and 12, one end of capacitor C in power pulse generator 4 is electrically connected to IGBT (Insulated Gate Bipolar Transistor). IGBT is divided into two paths: one path is electrically connected to one end of the first resistor R1, and the other path is electrically connected to relay group K. Relay group K is electrically connected to the first electrode 91 which serves as the positive terminal. The other end of the first resistor R1 and the other end of capacitor C are respectively electrically connected to one end of the second resistor R2, and the other end of the second resistor R2 is electrically connected to the second electrode 92.

The capacitor C stores high-voltage charge. The first resistor R1 and the second resistor R2 are the working loads across the relay group K, protecting the circuit. When high-voltage charge needs to be released to the electrodes, the circuit is switched on and off through the cooperation of IGBT (Insulated Gate Bipolar Transistor) and the relay group K. When one of the switches in the relay group K is closed, the IGBT conducts, releasing the high-voltage charge stored in the capacitor C through the connecting circuit to form a high-voltage pulse. When multiple pairs of electrodes need to generate corona simultaneously or sequentially, multiple energy storage capacitors and multiple relays are used to manage the switching of the circuit.

The power pulse generator 4 can set the voltage value (1000-8000V) and the pulse width value for releasing charge (1-200us) in the circuit.

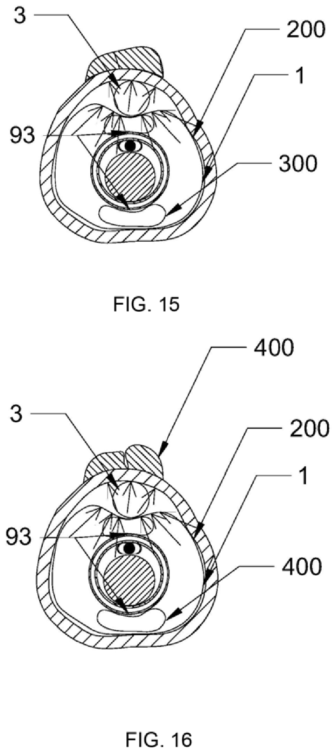

The present invention can be used to treat vascular stenosis and calcification. In use, under image guidance, the invention (shockwave balloon catheter) is introduced to the target site, i.e., the target vascular segment, through the guidewire 3. During introduction, the guidewire 3 only enters the balloon tip 5, not the balloon 1 or the catheter 2 (as shown in FIG. 14). Without electrolyte solution, the balloon 1 can smoothly pass through the stenosis and calcification area. Once balloon 1 reaches the target position, electrolyte solution is inflated into the balloon 1 through the catheter to a preset pressure, causing it to adhere to the vessel wall 200. At this time, guidewire 3 is pushed to adhere to the vessel wall, ensuring full contact between the balloon 1 and the lesion area. A pulsed high voltage is applied, causing the corona generator to produce corona discharge. The corona discharge phenomenon causes the electrolyte solution to generate vapor bubbles 300, compressing the electrolyte solution and continuously increasing the pressure inside the balloon 1, thereby transmitting the pressure to the target site and the guidewire 3 (as shown in FIG. 15). The localized high-stress area generated by stress concentration is conducive to focusing energy, which can be used to break up lesions 400 (calcification) by pushing or rotating (as shown in FIG. 16), improving the efficiency of calcification breaking. Stress concentration also brings greater local mechanical force, which can work in conjunction with the corona generator to break up calcified material and expand the vascular lumen. Depending on the degree of lesion and treatment needs, multiple corona discharges can be performed to ensure complete removal of the lesion. After each discharge, the balloon 1 can be slightly drained to change its diameter, and the position of the balloon dilation catheter can be adjusted to cover the entire lesion area. After treatment, the internal fluid is drained, the balloon 1 is retrieved, and the balloon dilation catheter is withdrawn from the body. Postoperative imaging examinations are performed to confirm vascular patency, thus achieving a good therapeutic effect. This approach can effectively break up calcium in calcified lesions in blood vessels through controllable instantaneous radial expansion of the balloon, preparing for subsequent vascular treatment.

The beneficial effects of the present invention are as follows.
1. In the prior art, the high temperature of the arc area during the arc discharge process can spread to the surrounding tissues, causing unnecessary thermal damage. However, the present invention uses corona discharge, which significantly improves the effectiveness, safety, ease of operation, reusability and precise control in the interventional treatment of calcified blood vessels.
2. At the same time, compared with the design of the guidewire inside the catheter, the external placement of the guidewire is more flexible during the operation. The operator can control the guidewire more directly, can more accurately control the advancement and retraction of the catheter, and can more effectively exert the cutting effect. When the corona generator generates mechanical stress outward, the guidewire generates stress concentration effect, which works together with the pulse catheter to facilitate the breaking of more stubborn calcifications.
3. External placement of the guidewire also increases the volume of electrolytes inside the balloon, which increases the current and the vapor bubble, generating greater mechanical stress and thus generating more energy, thereby improving the ability to break up and cut refractory calcifications in interventional treatment of calcified blood vessels.

## Claims

1. An externally guidewire shockwave balloon catheter, comprising:
a balloon (1);
a catheter (2); and
a guidewire (3), wherein the catheter (2) comprises an inner support rod (21) and an outer tube (22) that are deformable and arranged to support the balloon (1), wherein the inner support rod (21) is located inside the outer tube (22) and is coaxial with the outer tube (22), a gap is provided between the outer tube (22) and the inner support rod (21), an inner cavity of the balloon (1) is communicated with the gap to allow the introduction of electrolyte liquid, wherein a distal end of the inner support rod (21) is extended out from a distal end of the outer tube (22), a proximal end of the balloon (1) is sealed to the distal end of the outer tube (22), a distal end of the balloon (1) is provided with a balloon tip (5), the balloon (1) is sealed to the distal end of the inner support rod (21) through the balloon tip (5), a corona generator is provided at a position of the inner support rod (21) located at the balloon (1) to achieve corona discharge to cause the electrolyte liquid in the balloon (1) to have a corona reaction, so as to generate steam bubbles to expand the balloon (1), wherein a guide wire hole (7) is provided at a distal end of the balloon tip (5) to penetrate the distal end of the balloon tip (5), the guide wire hole (7) is not communicated to the inner cavity of the balloon (1), wherein an inlet (6) is provided on the balloon tip (5) to communicate with the guide wire hole (7) so as to allow the guide wire (3) to be inserted into the guide wire hole (7) through the inlet (6) and then extended out from the distal end of the balloon tip (5), so that a part of the guide wire (3) is placed outside the balloon (1) and the catheter (2), in addition to guiding the balloon (1), the guide wire (3) also serves as a cutting guide wire to assist in cutting the lesion site after the electrolyte liquid has a corona reaction.

2. The externally guidewire shockwave balloon catheter according to claim 1, wherein a diameter of the inner support rod (21) is less than or equal to a diameter of the inlet (6), and the diameter of the inner support rod (21) is less than or equal to a diameter of the guidewire hole (7).

3. The externally guidewire shockwave balloon catheter according to claim 2, wherein the corona generator comprises at least one set of electrode group (9) which is provided with discharge regions (96), where each set of electrodes (9) comprises at lease one first electrode (91) connected to a positive lead and at least one second electrode (92) connected to a negative lead, wherein the first electrode (91) and the second electrode (92) in the electrode group (9) are spaced apart, the first electrode (91) and the second electrode (92) are provided with discharge regions (96) that are in contact with the electrolyte liquid filled in the inner cavity of the balloon (1), so as to allow an electric field to be formed between the first electrode (91) and the second electrode (92), wherein the first electrode (91) and the second electrode (92) are fixed on the inner support rod (21).

4. The externally guidewire shockwave balloon catheter according to claim 3, wherein the positive and negative leads are respectively provided with exposed areas connected to the first electrode (91) and the second electrode (92), and the exposed areas are covered with metal electrode sleeves (94) which are respectively connected and fixed to the first electrode (91) and the second electrode (92).

5. The externally guidewire shockwave balloon catheter according to claim 4, wherein each of the first electrode (91) and the second electrode (92) has a ring shape, and a groove (95) is respectively formed on an edge of an inner wall of each of the first electrode (91) and the second electrode (92), and a metal electrode sleeve (94) is fixed on the groove (95).

6. The externally guidewire shockwave balloon catheter according to claim 5, wherein outer walls of the first electrode (91) and the second electrode (92) are respectively provided with a first insulating layer (912) and a second insulating layer (922) at other positions except for the discharge regions (96), so as to expose the discharge region (96).

7. The externally guidewire shockwave balloon catheter according to claim 6, wherein the first electrode (91) is provided with at least one first through hole (911), the second electrode (92) is provided with at least one second through hole (921), the at least one first through hole (911) and the at least one second through hole (921) respectively constitute the discharge regions (96), the first insulating layer (912) is completely covered on the rest position of the first electrode (91) except for the at least one first through hole (911), and the second insulating layer (922) is completely covered the rest position of the second electrode (92) except for the at least one second through hole (921), so as to form a directional electric field between the first electrode (91) and the second electrode (92).

8. The externally guidewire shockwave balloon catheter according to one of claims 3-7, wherein the at least one set of electrode group (9) comprises a single electrode group (9) which comprises one first electrode (91) and one second electrode (92).

9. The externally guidewire shockwave balloon catheter according to one of claims 3-7, wherein the at least one set of electrode group (9) comprises a single electrode group (9) which comprises two first electrodes (91) and one second electrode (92), wherein the second electrode (92) is disposed between the two first electrodes (91).

10. The externally guidewire shockwave balloon catheter according to one of claims 3-7, wherein the at least one set of electrode group (9) comprises two sets of electrodes (9), wherein one electrode set (9) comprises two first electrodes (91) and one second electrode (92), and the other electrode set (9) comprises one first electrode (91) and one second electrode (92).
